Europäisches Patentamt

European Patent Office

Office européen des brevets

(11) Publication number: **0 281 551 B1**

# EUROPEAN PATENT SPECIFICATION

(45) Date of publication of patent specification: **25.07.90**

(51) Int. Cl.⁵: **A 61 L 9/16**

(21) Application number: **86904755.5**

(22) Date of filing: **15.08.86**

(86) International application number:
**PCT/BR86/00014**

(87) International publication number:
**WO 88/01181 25.02.88 Gazette 88/05**

(54) **APPARATUS FOR THE OXIDATION OF PARTICLES SUSPENDED IN THE AIR.**

(43) Date of publication of application:
**14.09.88 Bulletin 88/37**

(45) Publication of the grant of the patent:
**25.07.90 Bulletin 90/30**

(84) Designated Contracting States:
**AT BE CH DE FR GB IT LI LU NL SE**

(56) References cited:
**FR-A-2 574 298**

(73) Proprietor: **CLOVER ELETRONICA LTDA.**
**Rua Mosela No. 1239-F**
**Petropolis, RJ (BR)**

(72) Inventor: **FIORENZANO, Alintor, Jr.**
**Rua 14 Bis No. 48**
**Petrópolis, RJ (BR)**

(74) Representative: **Nettleton, John Victor et al**
**Abel & Imray Northumberland House 303-306**
**High Holborn**
**London, WC1V 7LH (GB)**

Courier Press, Leamington Spa, England.

**Description**

The present invention refers to an apparatus for the oxidation of particles suspended in the air, comprising a plurality of ducts open at both ends and provided in a refractory block, within which ducts is disposed a resistive wire, said refractory material block being disposed within a box. Such an apparatus is known from FR—A—2 574 298.

Experiments have demonstrated that such an apparatus is viable only if some parameters are observed which are not considered in the prior art.

The present invention aims to provide an improvement of said apparatus, in order to permit a rapid oxidation of particles suspended in the air, under application of a relatively low total power and with high efficiency.

This aim is achieved by the invention due to the fact that each of said ducts has a maximum volume of $10^{-3} m^3$ and a maximum cross section area of 1 cm$^2$, the length and cross section being dimensioned in such a way that the minimum permanence time of the air mass within the ducts is 0,01 second and the minimum power density within the ducts is 50 KW/m$^3$, the box being provided with a fan having a motor which is controllable by an electronic control circuit connected to a thermal sensor disposed within the refractory material block to activate the motor according to the temperature of the block.

Experiments have shown that by maintaining the above parameters it is possible to achieve a sterilization with high efficiency and low costs in closed ambience.

The present invention provides a new way of achieving the oxidation of particles suspended in the air in ambience, should they be alive forms or inaminated, reducing also the relative humidity in a surprisingly efficient manner. The high oxidation rate, which is achieved inside the ducts, promotes the elimination of microorganisms from the air by means of the oxidation of the proteins as well as eliminates odors and oxidates some molecular forms of gases such as CO (toxicant) transforming it $CO_2$ (inert to human organism). It refers to an improvement of extreme importance on the asepsis methods, polution control, conservation of works of art, conservation of agricultural products and the like.

According to a preferred embodiment, the present invention comprises a refractory material block provided with more than a hundred cappilaries whereby a high power density is generated.

A preferred embodiment consists in executing the ducts as cylindrical perforations in a refractory block disposed inside a box, which is provided below the block. This embodiment will be described afterwards in more detail.

An embodiment example utilizes a refractory material block of 7 cm thickness in which 120 trespassing cylindrical ducts with about 2,0 to 2,5 mm diameter are provided, whereby a NiCr filament with a resistivity of 24 Ω/m is axially passed through the ducts, dissipating a total power of 500 W. The power dissipated in each duct is 3,5 W for a total length to the NiCr filament of 10 m.

The refractory material block has, preferably, the following weight composition: 45% $SiO_2$, 42% $Al_2O_3$, 4% $ZrO_2$, 1% $TiO_2$, 6% CaO and 2% MgO. This refractory material has a thermal conductivity coefficient K = 0,300 Kcal/h°C m/m$^2$ at a temperature of 600°C.

Effecting the calculation for this embodiment example, it is found;

a) The power density within each duct (up):

up = duct power/duct volume, where duct power = 3,5 W

$$\text{duct volume} = \frac{\pi D^2}{4} \times L = \pi \times \frac{(2 \times 10^{-3})^2}{4} \times 7 \times 10^{-2} m^3 = 2,2 \times 10^{-7} m^3$$

$$= 2,2 \times 10^{-7} m^3$$

up = $15,90 \times 10^6 W/m^3$, what represents an extraordinarily high value.

b) Energy density transformed within each duct (uEtd):

uEtd = up × t, where t is the average permanence time of the air mass within the duct. For t = 0,1s:

uEtd = $1,59 \times 10^6 J/m^3$

Considering the importance of the radiation in the infrared band in the oxidation process of particle:

c) Energy density in the infrared band (uEir):

uEir = $1,59 \times 10^4 J/m^3$,

assuming that 1% of uEtd is in the infrared band.

d) Energy of each photon generated with the wave length

$$\lambda_{max} (E_{max}): \quad E_{max} = \frac{h c}{\lambda_{max}}, \text{ where } \begin{array}{l} h = 6,63 \times 10^{-34} \text{ Js} \\ c = 3 \times 10^8 \text{ m/s} \\ \lambda_{max} = \dfrac{2,898 \times 10^{-3} \text{ m/°K}}{T} \end{array}$$

$$T = 500°C = 773°K$$

then $E_{max} = 6,63 \times 10^{-34} \times \dfrac{3 \times 10^8}{2,898 \times 10^{-3}/773}$

$E_{max} = 5,30 \times 10^{-20}$ J

2

e) Number of photons per cubic meter $(N\lambda_{max})$:

$N\lambda_{max} = 3,00 \times 10^{23}$ photons/m$^3$, which is a rather high number.

Associating those results to the scientific knowledge, one verifies that the air mass carrying suspended particles has those particles oxidated in an undoubtful way when submitted to conditions of thermal power and infrared power together with the Nickel of the filament which acts as a catalyst.

The control of the air flow through the ducts, has the objective of making the permanence time of the air mass as well as the temperature within the ducts not affected by the environment conditions. This is done by means of a signal generated by a Constantan Iron sensor, i.e., a thermocouple (other kinds of thermal sensors can be used) which generates a control signal by means of an electronic circuit whose outlet controls the air flow of a fan.

The apparatus will be described, based on the embodiment example represented on the drawings. The figures show schematically:

figure 1 a side sectioned view of the apparatus;

figure 2 the basic electric circuit of the apparatus of figure 1;

figure 3 a graphic of an experiment carried by the Brazilian Ministry of Health.

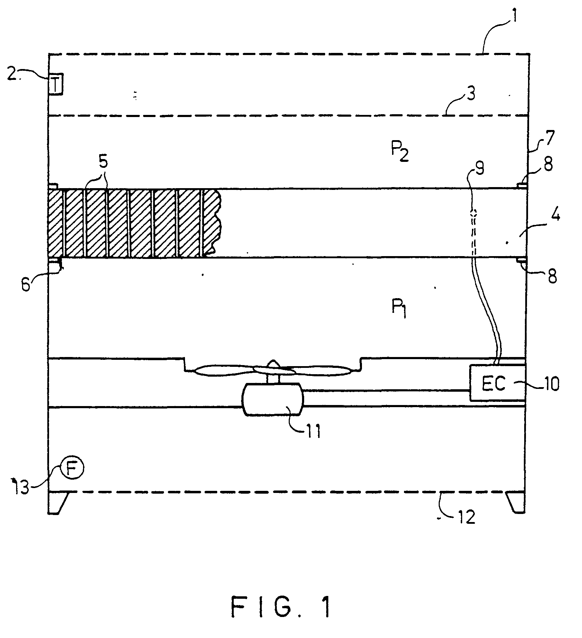

The apparatus represented in figure 1 is disposed within a metal box 7 provided with superior 1 and inferior 12 protecting network screen. The refractory block 4, within which are executed the small volume ducts 5, is fixed to the box 7 by fixing pieces 8. The piece 6 serves for the fixation of the electric wire, which is passed through the ducts 5 in a tensive way in the art of a "needle-work", the wire not being represented in figure 1 in order to simplify it.

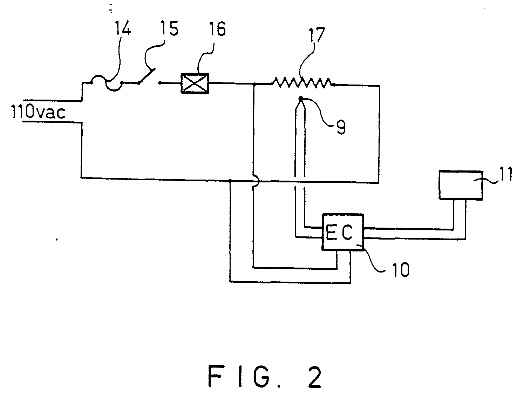

The electronic control circuit 10 is connected to a thermal sensor 9 which is allocated within the block 4, although it is also possible to dispose the sensor on the outlet of the ducts. The control system of the fan motor 11 can be executed as a continuous control, but in this example it is executed as an ON-OFF type with hysterisis, as represented in figure 2.

A perforated inox steel 3, provided with perforations with a diameter of about 2 mm, is fixed within the box 7 above the block 4, in order to actuate as heat exchanger, cooling the air that leaves the box, a thermostate or thermal circuit breaker 2 being disposed above the perforated plate 3 to turn off the apparatus if the temperature of the air leaving the box 7 exceeds a predetermined limit.

A fuse 13 guarantees the apparatus against possible overload.

On the scheme represented on figure 2 there are provided a Slow-Blow type fuse 14 of 5 amperes, an ON-OFF type switch 15, a thermostate 16 with disarm at 70°C, a Ni-Cr filament 17 of 24,2 $\Omega$/m $\times$ 500 W for the 110 V version, a thermal sensor 9 of the thermocouple type, as well as the fan's motor 11 and the electronic control circuit 10.

The electronic control circuit 10 operates in such a way, that when it receives a signal from the thermal sensor 9 indicating that the temperature on the block 4 reached a predetermined maximum value, it activates the fan's motor 11, increasing the pressure P1 below the block and, thus, increasing the air flow speed through the ducts, the pressure P2 above the block being smaller than P1. The raise of air flow causes the temperature to fall until it reaches a predetermined minimum value, when, then, the electronic control circuit 10 deactivates the fan. With this system it is possible to maintain the temperature and the permanence time of the air within the ducts within predetermined values.

A model based on the above described example supplied a mean flow of 35 m$^3$/h of sterilized air for a room temperature of 30°C and relative humidity of 85%.

The temperature variation, in its maximum and minimum spots through the ducts, was of 95°C in the inferior portion, 450°C at 6 cm above the inferior portion and 300°C on the outlet of the ducts at 7 cm.

The model proved to be efficient in the reduction of biological activity in the air in a room of 250 cubic meters volume. Thus, it was verified that the present invention does not heat significantly the room, being destinated only to the air treatment, reducing the relative humidity and the biological activity.

For instance, it may be mentioned that the average thermal irradiation of a grown-up person is about 100 Watts. Therefore the 500 W model of the present invention is thermically equivalent to 5 people within a room of about 100 square meters and 2,5 meter high. This demonstrates that its load on the cooling systems is insigificant, thus being of great utility in rooms where there is no air renovation.

Since it does not produce a significant heating of the room, due to its low power per air volume unit relation, the present invention independs on the climatization form and can be used indiscriminately.

In an experiment carried in Bremen University (German Federal Republic) with an apparatus of the present type inside a garbage room of 21,2 m$^3$ (3,67 m $\times$ 2,88 m $\times$ 2,00 m), the following results were found:

parameters of experiment:

operation time of the apparatus: 64 h

number of apparatus: 2

results table:

| germs content | before | after | reduction |
|---|---|---|---|
| total germs content | 14 | 10 | 28% |
| yeast + mould | 5 | 1 | 80% |
| enterobacteriacese | 4 | — | 100% |
| bac. cereus | 15 | 3 | 80% |

encubating time: 7 days

Conclusions: each apparatus could reduce, e.g., the yeast + incidence in 80% for a room of 10 m³. In the gross, it seems that the apparatus used in the experiment permit an advantageous germ elimination effect.

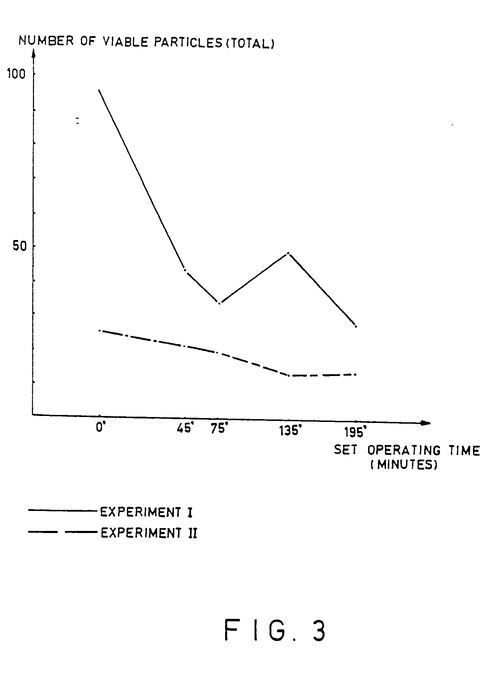

Yet, another experience carried in the Brazilian Ministry of Health — Oswaldo Cruz Foundation — National Institute for Quality Control in Health resulted in a particle reduction as represented on figure 3. The parameters of the experiment were as follows:

specification of se: flow = 9 m³/h

volume of the test room: 25,80 m³

number of sterilizers: 3

experimental method: growing of colonies of microorganisms (fungi and bacteria) in Petri dishes, in blood agar and Sabourand's agar media exposed to the environment for 15 minutes.

The process is repeated with different set operating periods (0 min, 45 min, 75 min, 135 min and 195 min).

Conclusions and remarks were stated as follows:

The exponential pattern $e^y = e^{93,106} \cdot x^{-11,83}$ is appropriate for representing the decrease of viable particles with set operating time.

Both factors, set operation time and also the day the experiments were carried out are significant. We should interpret the difference observed as a consequence rather of the (successive) day than of the fact that the experimenter remained in the room or circulated entering and leaving it.

Considering that the experiments were carried out on succeeding days, a large decrease evident in the number of viable particles obtained in experiment II, and such difference is statistically significant.

As can be seen from the graph, the number of particles remains much stabler, though decreasing with time, on the second day, so that there is a stabilization of the general level of particles in the environment. The difference between the experiments (days) proves highly significant in the beginning (0') and at 135'. In the latter case it is likely that a variation has occurred in the voltage, causing the number of particles to increase, contrary to expectation.

The significance of the interaction is reflected by the fact that the variations observed between set operation times depended on the day (1st or 2nd) the experiment was performed.

Accordingly, we may conclude that the general level of particles in the environment tends to vary in function of the initial concentration of particles. On the second day, the concentration is much smaller (a highly significant difference within $t_1 = 0'$ and the number of particles decreases with operation time, but not so sharply.

As a result of the general analysis applied, we conclude that the set is efficient in reducing the number of viable particles in the environment.

So the object in a second stage of the analysis, would then be to find out whether the reduction of the number of particles attains that minimum level of concentration which characterizes a sterile environment.

According to N.A.S.A. specifications, a sterile environment must contain no more than 0.0035 viable particles per liter.

Since the flow in the set is 9 cubic meters per hour and the volume of the room 25.80 cubic meters, we conclude that sterilization of the room would occur after approximately 2 hours 52 minutes or 172 minutes of set operation.

The present experiment used three (3) sets operating simultaneously, so room sterilization time would be reduced to 172 minutes divided by three, or 57 minutes, i.e. about one hour.

Considering the volume above the area where the Petri dishes were exposed to be approximately one cubic meter, we conclude that an index of 3.5 viable particles per cubic meter would represent a correct index of sterility to the environment.

In experiment I, the values were significant at the 5% level with 75' and 135', and almost significant (but that is no longer significant) at 195'; accordingly, one concludes that on that first day, when the room had an initial natural concentration of particles in the environment, *after 3 hours of operation* was the set able to reduce the environmental air to a level considered sterile.

In experiment II, i.e. on the second day, the differences were not significant, with much lower concentration, and in this case the atmosphere may be considered sterile.

4

# EP 0 281 551 B1

**Claims**

1. Apparatus for the oxidation of particles suspended in the air, comprising a plurality of ducts open at both ends and provided in a refractory material block (4), within which ducts (5) is disposed a resistive wire, said refractory material block being disposed within a box, characterized in that each of said ducts (5) has a maximum volume of $10^{-3} m^3$ and a maximum cross section area of 1 cm$^2$, the length and cross section being dimensioned in such a way that the minimum permanence time of the air mass within the ducts (5) is 0,01 second and the minimum power density within the ducts is 50 KW/m$^3$, the box (7) being provided with a fan having a motor (11) which is controllable by an electronic control circuit (10) connected to a thermal sensor (9) disposed within the refractory material block (4) to activate the motor (11) according to the temperature of the block.

2. Apparatus according to claim 1, characterized in that said ducts (5) have a maximum volume of $5 \times 10^{-5} m^3$ and a maximum cross section area of 0,1 cm$^2$, the minimum permanence time of the air within the ducts (5) being equal to 0,1 second and the minimum power density within the ducts being equal to 250 KW/m$^3$.

3. Apparatus according to claim 1, characterized in that said ducts (5) have a cross section area of 3 to 5 mm$^2$ and a length of about 7 cm.

4. Apparatus according to claim 1 or 3, characterized in that said ducts (5) are cylindrical having a round cross section with a diameter of 2,0 to 2,5 mm.

5. Apparatus according to claim 1, characterized in that an only NiCr wire is axially passed through all ducts (5), said wire having a relative resistivity of about 24,2 $\Omega$/m.

6. Apparatus according to any preceding claim, characterized in that the refractory material of said block (4) has a weight compositon of 45% $SiO_2$, 42% $Al_2O_3$, 4% $ZrO_2$, 1% $TiO_2$, 6% $CaO$ and 2% $MgO$.

7. Apparatus according to any preceding claim, characterized in that the electronic control circuit (10) comprises an ON-OFF type control to activate and desactivate the motor (11) of the fan, the electronic control circuit (10) being able to activate the motor (11) of the fan when the temperature on the refractory material block (4) reaches a predetermined maximum value and to desactivate it when said temperature decreases to a predetermined minimum value.

8. Apparatus according to any one of claims 1 to 6, characterized in that the electronic control circuit (10) comprises a continuous type control to activate the motor (11) of the fan in various speeds depending on the temperature of the refractory material block (4).

9. Apparatus according to any preceding claim, characterized in that said box (7) comprises a perforated plate (3) disposed above the refractory material block (4), said perforated plate (3) being provided with a plurality of perforations with a diameter of about 2 mm.

**Patentansprüche**

1. Vorrichtung zum Oxidieren von Teilchen in Luft mit einer Vielzahl von an beiden Enden offenen Kanälen in einem Block (4) aus hitzebeständigem Material, wobei in den Kanälen (5) ein Widerstandsdraht angeordnet ist, dadurch gekennzeichnet, daß der genannte hitzebeständige Materialblock in einem Gehäuse angeordnet ist, daß jede der genannten Kanälen (5) ein maximales Volumen von $10^{-3} m^3$ aufweist und einen maximalen Querschnitt von 1 cm$^2$, wobei die Länge und der Querschnitt so bemessen sind, daß die minimale Aufenthaltszeit der Luftmasse in den Kanälen (5) 0,01 sec und die minimale Leistungsdichte in den Kanälen 50 KW/m$^3$ beträgt, und das Gehäuse (7) mit einem Gebläse ausgerüstet ist, dessen Motor (11) durch eine elektronische Steuereinheit (10) steuerbar ist, die mit einem thermischen Sensor (9) verbunden ist, der im hitzebeständigem Materialblock (4) angeordnet ist, um den Motor (11) entsprechend der Temperatur des Blocks zu betätigen.

2. Vorrichtung nach Anspruch 1, dadurch gekennzeichnet, daß die genannten Kanälen (5) ein maximales Volumen von $5 \times 10^{-5} m^3$ aufweisen und einen maximalen Querschnitt von 0,1 cm$^2$, wobei die minimale Aufenthaltszeit von Luft in den Kanälen (5) bei 0,1 sec liegt und die minimale Leistungsdichte in den Kanälen bei 250 KW/m$^3$ liegt.

3. Vorrichtung nach Anspruch 1, dadurch gekennzeichnet, daß die genannten Kanälen (5) eine Querschnittsfläche von 3 bis 5 mm$^2$ und eine Länge von etwa 7 cm haben.

4. Vorrichtung nach einem der Ansprüche 1 bis 3, dadurch gekennzeichnet, daß die genannten Kanälen (5) zylinderförmig sind mit rundem Querschnitt, dessen Durchmesser im Bereich von 2,0 bis 2,5 mm liegt.

5. Vorrichtung nach Anspruch 1, dadurch gekennzeichnet, daß ein aus NiCr bestehender Draht axial durch die Kanälen (5) greift, wobei der Draht einen relativen Widerstand von 24,2 Ohm/m aufweist.

6. Vorrichtung nach einem der vorangehenden Ansprüche, dadurch gekennzeichnet, daß der hitzebeständige Materialblock (4) eine Gewichtszusammensetzung von 45% $SiO_2$, 42% $Al_2O_3$, 4% $ZrO_2$, 1% $TiO_2$, 6% $CaO$ und 2% $MgO$ hat.

7. Vorrichtung nach einem der vorangehenden Ansprüche, dadurch gekennzeichnet, daß die elektronische Steuereinrichtung (10) eine AN/AUS-Steuerung aufweist, um den Motor (11) des Gebläses zu betätigen bzw. in Stillstand zu setzen, wobei der elektronische Steuerkreis (10) geeignet ist, den Motor (11) des Gebläses zu betätigen, wenn die Temperatur auf dem hitzebeständigen Materialblock (4) einen vorgegebenen maximalen Wert erreicht, während eine Stillegung des Motors erfolgt, wenn die Temperatur unter einen vorgegebenen Minimalwert fällt.

8. Vorrichtung nach einem der Ansprüche 1 bis 6, dadurch gekennzeichnet, daß die elektronische Steuereinheit (10) eine kontinuierliche Steuerung ermöglicht, um unterschiedliche Geschwindigkeiten des Motors (11) in Abhängigkeit von der Temperatur des hitzebeständigen Materialblockes (4) einzustellen.

9. Vorrichtung nach einem der vorhergehenden Ansprüche, dadurch gekennzeichnet, daß das genannte Gehäuse (7) eine durchlöcherte Platte (3) aufweist, die oberhalb des hitzebeständigen Materialblockes (4) angeordnet ist und mit einer Vielzahl von Perforationen versehen ist, die einen Durchmesser von etwa 2 mm aufweisen.

## Revendications

1. Appareil pour l'oxydation de particules suspendues dans l'air, comprenant une pluralité de conduits ouverts à leurs deux extrémités et prévus dans un bloc (4) en matière réfractaire, un fil résistant étant disposé à l'intérieur desdits conduits (5), ledit bloc en matière réfractaire étant disposé à l'intérieur d'une boîte, caractérisé en ce que chacun desdits conduits (5) a un volume maximal de $10^{-3}m^3$ et une surface de section transversale maximale de 1 cm², la longueur et la section transversale étant dimensionnées de telle mainère que le temps de séjour minimal de la masse d'air se trouvant à l'intérieur des conduits (5) soit de 0,01 seconde et que la densité minimale de puissance à l'intérieur des conduits soit de 50 kW/m³, la boîte (7) étant munie d'un ventilateur comportant un moteur (11) qui peut être commandé par un circuit (10) de commande électronique relié à un détecteur thermique (9) disposé à l'intérieur du bloc en matière réfractaire (4) afin d'activer le moteur (11) en fonction de la température du bloc.

2. Appareil selon la revendication 1, caractérisé en ce que lesdits conduits (5) ont un volume maximal de $5 \times 10^{-5}m^3$ et une surface de section transversale maximale de 0,1 cm², le temps de séjour minimal de l'air se trouvant à l'intérieur des conduits étant égal à 0,1 seconde et la densité de puissance minimale à l'intérieur des conduits étant égale à 250 kW/m³.

3. Appareil selon la revendication 1, caractérisé en ce que lesdits conduits (5) ont une surface de section transversale de 3 à 5 mm² et un longueur d'environ 7 cm.

4. Appareil selon la revendication 1 ou 3, caractérisé en ce que lesdits conduits sont cylindriques et présentent une section transversale circulaire avec un diamètre de 2,0 à 2,5 mm.

5. Appareil selon la revendication 1, caractérisé en ce qu'un seul fil en Ni Cr est passé axialement à travers tous les conduits, ledit fil ayant une résistivité relative d'environ 24,2 ohm/m.

6. Appareil selon l'une quelconque des revendications précédentes, caractérisé en ce que la matière réfractaire dudit bloc (4) a une composition pondérale de 45% $SiO_2$, 42% $Al_2O_3$, 4% $ZrO_2$, 1% $TiO_2$, 6% CaO et 2% MgO.

7. Appareil selon l'une quelconque des revendications précédentes, caractérisé en ce que le circuit (10) de contrôle électronique comprend une commande de type tout ou rien pour activer et désactiver le moteur (11) du ventilateur, le circuit (10) de commande électronique pouvant activer le moteur (11) du ventilateur lorsque la température sur le bloc (4) de matière réfractaire atteint une valeur maximale prédéterminée et le désactiver lorsque ladite température décroit jusqu'a une valeur minimale prédéterminée.

8. Appareil selon l'une quelconque des revendications 1 à 6, caractérisé en ce que le circuit (10) de commande électronique comprend une commande de type continu pour activer le moteur (11) du ventilateur à des vitesses différentes en fonction de la températurre du bloc (4) de matière réfractaire.

9. Appareil selon l'une quelconque des revendications précédentes, caractérisé en ce que ladite boîte (7) comprend une plaque perforée (3) disposée au-dessus du bloc (4) de matière réfractaire, ladite plaque perforée (3) étant munie d'une pluralité de perforations ayant un diamètre d'environ 2 mm.

FIG. 1

FIG. 2

NUMBER OF VIABLE PARTICLES (TOTAL)

SET OPERATING TIME
(MINUTES)

———————— EXPERIMENT I
——— ——— EXPERIMENT II

F I G. 3